(19) Europäisches Patentamt
European Patent Office
Office européen des brevets

(11) Publication number: **0 339 285 A2**

(12) **EUROPEAN PATENT APPLICATION**

(21) Application number: **89105626.9**

(22) Date of filing: **30.03.89**

(51) Int. Cl.4: **C12P 21/02 , C12N 15/00 , C12N 1/20 , C12N 5/00 , //(C12N1/20,C12R1:19,1:125, 1:91)**

The applicant has filed a statement in accordance with Rule 28 (4) EPC (issue of a sample only to an expert). Accession number(s) of the deposit(s): ATCC 67916

(30) Priority: **31.03.88 US 176802**

(43) Date of publication of application: **02.11.89 Bulletin 89/44**

(84) Designated Contracting States: **AT BE CH DE ES FR GB GR IT LI LU NL SE**

(71) Applicant: **RORER INTERNATIONAL (OVERSEAS) INC. (a Delaware corporation)**
**P.O. Box 145**
**Lewes Delaware 19958(US)**

(72) Inventor: **Kaplan, Ruth**
**1505 Cotswald Court**
**West Chester Pennsylvania(US)**
Inventor: **Jaye, Michael**
**142 Lynwood Avenue**
**Glenside Pennsylvania(US)**

(74) Representative: **Vossius & Partner**
**Siebertstrasse 4 P.O. Box 86 07 67**
**D-8000 München 86(DE)**

(54) **Recombinant endonexin II.**

(57) Endonexin II, a novel inhibitor of coagulation and phospholipase $A_2$, is disclosed. Also disclosed are its recombinant DNA directed synthesis and clinical use as an anti-inflammatory.

EP 0 339 285 A2

# RECOMBINANT ENDONEXIN II

This invention relates to the field of structural gene cloning and the use of such genes in the recombinant DNA directed synthesis of desired protein products. More particularly, it relates to the calcium and phospholipid binding protein endonexin II, a novel inhibitor of coagulation and the function of phospholipase $A_2$, its recombinant DNA directed synthesis and use clinically for the treatment of various conditions where an anti-inflammatory effect is desired without the adverse side effects of steroids or where inhibition of blood coagulation is desired.

In general, recombinant DNA techniques are known. See Methods in Enzymology, (Academic Press) Volumes 65 and 68 1979); 100 and 101 (1983), and the references cited therein, all of which are incorporated herein by reference. An extensive technical discussion embodying most commonly used recombinant DNA methodologies can be found in Maniatis, et al., Molecular Cloning, Cold Spring Harbor Laboratory (1982). Genes coding for various polypeptides may be cloned by incorporating a DNA fragment coding for the polypeptide in a recombinant DNA vehicle, e.g., plasmid or viral vectors, introducing this into a suitable host, typically Escherichia coli (E. coli), and identifying colonies or plaques containing the desired DNA fragment. Such clones may be grown and used to produce the desired polypeptide on a large scale.

Several groups of workers including our own have isolated mixtures of messenger RNA (mRNA) from eukaryotic cells and employed a series of enzymatic reactions to synthesize double stranded DNA copies which are complementary to this mRNA mixture. In the first reaction, mRNA is transcribed to form a single-stranded complementary DNA (cDNA) by an RNA-directed DNA polymerase, also called reverse transcriptase. Reverse transcriptase synthesizes DNA in the 5′-3′ direction, utilizes deoxyribonucleoside 5′-triphosphates as precursors, and requires both a template and primer strand, the latter of which must have a free 3′-hydroxyl terminus. Reverse transcriptase products, whether partial or complete copies of the mRNA template, often possess short, partially double-stranded hairpins ("loops") at their 3′ termini. In the second reaction, these "hairpin loops" can be exploited as primers for DNA polymerases. Preformed DNA is required both as a template and as a primer in the action of DNA polymerase. The DNA polymerase requires the presence of a DNA strand having a free 3′-hydroxyl group, to which new nucleotides are added to extend the chain in the 5′ to 3′ direction. The products of such sequential reverse transcriptase and DNA polymerase reactions still possess a loop at one end. The apex of the loop or "fold-point" of the double-stranded DNA, which has thus been created, is substantially a single-strand segment. In the third reaction, this single-strand segment is cleaved with the single strand specific nuclease S1 to generate a "blunt-end" duplex DNA segment. This general method is applicable to any mRNA mixture, and is described by Buell, et al., J. Biol. Chem., 253:2483 (1978).

The resulting double-stranded cDNA mixture (ds-cDNA) is inserted into cloning vehicles by any one of many known techniques, depending at least in part on the particular vehicle used. Various insertion methods are discussed in considerable detail in Methods in Enzymology, 68:16-18, 1980, and the references cited therein.

Once the DNA segments are inserted, the cloning vehicle is sued to transform a suitable host. These cloning vehicles usually impart an antibiotic resistance trait on the host. Such hosts are generally prokaryotic cells. At this point, only a few of the transformed or transfected hosts contain the desired cDNA. The sum of all transformed or transfected hosts constitutes a gene "library". The overall ds-cDNA library created by this method provides a representative sample of the coding information present in the mRNA mixture used as the starting material

If appropriate oligonucleotide sequences are available, as in this invention, they can be used to identify clones of interest as follows. Individual transformed or transfected cells are grown in colonies on a nitrocellulose filter or are infected with recombinant bacteriophage. DNA from the colony or phage plaques is denatured, bound to the filter by heating, and the filter is then incubated with a labeled oligonucleotide probe. In this invention, the oligonucleotide sequences are complementary to the endonexin II gene. The probe hybridized with the denatured cDNA strand to which it is complementary and this is identified by autoradiography. The clones are characterized in order to identify a clone containing all the structural information for the desired protein. The nucleic acid sequence coding for the protein endonexin II is isolated and reinserted into an expression vector. The expression vector brings the endonexin II gene under the regulatory control of a specific prokaryotic or eukaryotic control element which allows the efficient expression (transcription and translation) of the cloned full length ds-cDNA. Thus, this general technique is only applicable to those proteins for which at least a portion of their amino acid or DNA sequence is known and for which an oligonucleotide probe is available. See, generally, Maniatis, et al., supra.

More recently, methods have been developed to identify specific clones by probing bacterial colonies or phage plaques with antibodies specific for the encoded protein of interest. This method, employed in this invention, can only be used with "expression vector" cloning vehicles since elaboration of the protein product is required. The structural gene is inserted into the veotor adjacent to the regulatory gene, and introduced into cells. The cells are then lysed, either by the vector or by chemical methods, and the protein is detected by a specific antibody and a labelling system, e.g., enzyme immunoassay. An example of this is the lambda gt11 system described by Young and David, Proc. Nat'l. Acad. Sci. USA, 80: 1194-1198 (1983) and Young and Davis, Science, 22:778 (1983) .

Lipocortins are a family of related proteins that have been detected in a number of tissues and share the property of being steroid-induced inhibitors of phospholipase $A_2$, ($PLA_2$). It is proposed that lipocortins mediate the anti-inflammatory effects of steroids by inhibiting the $PLA_2$-catalyzed formation of arachidonate and lysophosphatide from phospholipids. The released arachidonate is the precursor for the biosynthesis of prostaglandins and leukotrienes.

Two members of the lipocortin family, lipocortin I and II, have been isolated and their complete primary amino acid sequence has been deduced from cDNA clones (Wallner, B.P., et al. Nature [London] 320:77-81, 1986; Huang, K.S., et al., Cell 46:191-199, 1986; Saris, C.J.M., Cell 46:201-212, 1986). These two proteins share approximately 50% sequence identity. Lipocortins I and II have also been termed calpactin II and I, respectively (Glenney, J., Proc. Nat'l. Acad. Sci. USA, 83:4258-4262, 1986).

Herein, the isolation from human placenta of a novel inhibitor of phospholipase $A_2$ activity is described. We have named this protein "endonexin II". The entire primary amino acid sequence of this protein has been deduced from the nucleotide sequence of cDNA clones. It share approximately 50% sequence identity with lipocortins I and II. Endonexin II, or a derivative thereof, may be useful clinically for the treatment of various conditions where an anti-inflammatory effect is desired without the adverse side effects of steroids. Endonexin II could also be useful in the diagnosis and treatment of a number of other clinical conditions, especially those in which it is desirable to interfere with normal phospholipid structure or function, such as in blood coagulation.

The present invention has made it.possible to provide readily available, large quantities of endonexin II. This has been achieved through the use of rabbit antibodies directed against human endonexin II as well as with oligonucleotides whose design was based upon knowledge of a portion of the amino acid sequence of the protein. Production of endonexin II is achieved through the application of recombinant DNA technology to prepare cloning vehicles encoding endonexin II and procedures for recovering endonexin II essentially free of other proteins of human origin.

Accordingly, the present invention provides human endonexin II essentially free of other proteins of human origin. Endonexin II is produced by recombinant DNA techniques in host cells or other self-replicating systems and is provided in essentially pure form.

The invention further provides replicable expression vectors containing a DNA sequence encoding human endonexin II and host cell or cell-free self-replicating systems transformed or transfected thereby. The host system is usually prokaryotic, e.g, E. coli or B. subtilis, or eukaryotic cells.

Human endonexin II is produced by a process which is comprised of (a) preparing a replicable expression vector capable of expressing the DNA sequence encoding endonexin II in a suitable host cell or cell-free replicating system; (b) transforming said host system to obtain a recombinant host system; (c) maintaining said recombinant host system under conditions permitting expression of said endonexin II encoding DNA sequence to produce human endonexin II protein; and (d) recovering said human endonexin II protein. Preferably, the endonexin II encoding replicable expression vector is made by preparing a double stranded complementary DNA (ds-cDNA) representative of endonexin II and incorporating this into replicable expression vectors. The replicable expression vector is capable , in a cell or cell-free self-replicating recombinant system, of expressing human endonexin II. Examples of such eukaryotic systems include, but are not limited to, the utilizing of fragments of Epstein Barr Virus or Bovine Papilloma Virus, as well as systems based on the co-amplification of integrated genes, linked to the dihydrofolate reductase gene (dhfr) in appropriate cells such as Chinese hamster ovary (CHO) mutant cells.

In the accompanying drawings,

Figure 1 illustrates the completed nucleotide sequence of endonexin II cDNA.

Figure 2 illustrates the sequence identity between endonexin II and other members of the annexin family.

Figure 3 illustrates Northern blot analysis of poly(a)-containing RNA from Helcells (lane B), human placenta (lane C) and human umbilical vein endothelial cells (lane D). RNA size markers are in lane A.

As used herein, "human endonexin II" denotes human endonexin II produced by cell or cell-free culture systems, in bioactive forms having the capacity to inhibit the function of phospholipase $A_2$.

Different alleles of endonexin II may exist in nature. These variations may be characterized by difference (s) in the nucleotide sequence of the structural gene coding for proteins of identical biological function. In addition, the location and degree of post-translational modifications may vary and will depend to a degree upon the nature of the host and environment in which the protein is produced. It is possible to produce analogs having single or multiple amino acid substitutions, deletions, additions, or replacements. All such allelic variations, modifications, and analogs resulting in derivatives or human endonexin II which retain the biologically active properties of native human endonexin are included within the scope of this invention. This includes analogs of endonexin II with protein or nucleic acid sequences different than endonexin II, but which perform essentially the same biological functions.

"Expression vectors" refer to vectors which are capable of transcribing and translating DNA sequences contained therein, where such sequences are linked to other regulatory sequences capable of effecting their expression. These expression vectors must be replicable in the host organisms or systems either as episomes, bacteriophage, or as an integral part of the chromosomal DNA. One form of expression vector useful in recombinant DNA technology is the plasmid - an unintegrated (extrachromosomal), double-stranded DNA circle. Any other form of expression vector which serves an equivalent function is suitable for use in the process of this invention.

Recombinant vectors and methodology disclosed herein are suitable for use in host cells covering a wide range of prokaryotic and eukaryotic organisms. Prokaryotic cells are preferred for the cloning of DNA sequences and in the construction of vectors. For example, E. coli K12 strain HB101 (ATCC No. 33694), is particularly useful. Of course, other microbiol strains may be used: vectors containing replication and control sequences which are derived from species compatible with the host cell or system are used in connection with these strains. The vector ordinarily carries an origin of replication, as well as characteristics capable of providing phenotypic selection in transformed cells. For example, E. coli can be transformed using the vector pBR322, which contains genes for ampicillin and tetracycline resistance (Bolivar, et al., Gene, 2:95 [1977]).

These antibiotic resistance genes provide a means of identifying transformed cells. The expression vector may also contain control elements which can be used for the expression of the gene of interest. Common prokaryotic control elements used for expression of foreign DNA sequences in E. coli include the promoters and regulatory sequences derived from the lactose (lac) and tryptophan (trp) operons of E. coli, as well as the pR and pL promoters of bacteriophage lambda. Combinations of these elements have also been used (e.g., tac, which is a fusion of the trp promoter with the lactose operator has been especially useful in this laboratory). Other promoters have also been discovered and utilized, and details concerning their nucleotide sequences have been published enabling a skilled worker to combine and exploit them functionally.

In addition to prokaryotes, eukaryotic microbes, such as yeast cultures, may also be used. Saccharomyces cerevisiae, or common baker's yeast, is the most commonly used among eukaryotic microorganisms, although a number of other strains are commonly available. Suitable promoter sequences in yeast vectors include the promoters of 3-phosphoglvcerate kinase or other glycolytic enzymes. Suitable expression vectors may contain signals which provide for the polyadenylation and termination of the cloned gene's mRNA. Any vector containing a yeast-compatible promoter, origin of replication, and appropriate termination signals is suitable for expression of endonexin II.

In addition to microorganisms, cultures of cells derived from multicellular organisms may also be used as hosts. In principle, any such cell culture is workable, whether from a vertebrate or invertebrate source. However, interest has been greatest in vertebrate cells, and propagation of vertebrate cells in culture (tissue culture) has become a routine procedure in recent years. Examples of such useful hosts are the VERO, HeLa, HepG2, mouse C127, Chinese hamster ovary (CHO), BHK, COS-7, and MDCK cell lines. Expression vectors for such cells ordinarily include an origin or replication, a promoter located in front of the gene to be expressed, a translation start site, RNA splice sites, polyadenylation site, and transcriptional termination signals.

For use in mammalian cells, the control functions on the expression vectors are often provided by viral material. For example, commonly used promoters are derived from polyoma, Adenovirus 2, and most frequently, Simian Virus 40 (SV40). Further, it is also possible, and often desirable, to utilize promoter or control sequences naturally associated with the desired gene, provided such control sequences are compatible with the host system. To increase the rate of transcription, eukaryotic enhancer sequences can also be added to the construction. These sequences can be obtained from a variety of animal cells or oncogenic retroviruses such as the mouse sarcoma virus.

An origin of replication may be provided either by construction of the vector to include an exogenous origin, such as that provided by SV40 or other viral sources or may be provided by the host cell chromosomal replication mechanism. If the vector is integrated into the host cell chromosome, the latter is often sufficient.

Recombinant host cells are those which have been transformed with vectors constructed using recombinant DNA techniques. As defined herein, endonexin II is produced as a consequence of this transformation. Endonexin II produced by such cells is referred to as "recombinant endonexin II".

The procedures below are but some of a wide variety of well-established procedures utilized to produce specific reagents which are important to this invention. The general procedure for obtaining a messenger RNA (mRNA) mixture is to prepare an extract from a tissue sample (placenta, in this invention) or to culture cells producing the desired protein, and to extract the mRNA by a process such as that disclosed by Chirgwin, et al., Biochemistry, 18:5294 (1979). The RNA is enriched for poly(A)-containing mRNA by chromatography on oligo (dT) cellulose or poly(U) Sepharose, followed by elution of the poly(A)-containing mRNA-enriched fraction.

The above poly(A)-containing mRNA-enriched fraction is used to synthesize single-strand complementary cDNA (ss-cDNA) using reverse transcriptase. As a consequence of DNA synthesis, a hairpin loop is formed at the 3′end of the DNA which will initiate second strand DNA synthesis. Under appropriate conditions, this hairpin loop is used to effect synthesis of the ds-cDNA in the presence of DNA polymerase and deoxyribonucleoside triphosphates.

The resultant double-strand (ds-cDNA) is inserted into an expression vector by any one of many known techniques. In general, methods, etc., can be found in Maniatis, et al., supra, and Methods in Enzymology, Volumes 65 and 68 (1980); and 100 and 101 (1983). In general, the vector is linearized by at least one restriction endounuclease, which will produce at least two blunt or cohesive ends. The ds-cDNA is ligated into the vector insertion site.

If prokaryotic cells or other cells which contain substantial cell wall material are employed, the most common method of transformation with the expression vector is calcium chloride pretreatment [Cohen, R.N., et al. Proc. Nat'l. Acad. Sci. USA, 69:2110(1972)]. If cells without cell wall barriers are used as host cells, transfection is carried out by the calcium phosphate precipitation method [Graham and Van der Eb, Virology, 52:456 (1973)].

Clones containing the endonexin II gene are identified using as probes the $^{32}P$ cDNA inserts from endonexin II recombinants isolated during the initial screening of the recombinant lambda gt11 cDNA library with endonexin II - specific oligonucleotides, and/or with antibody directed against endonexin II protein. Nucleotide sequence techniques are then used to determine the sequence of the cDNA and ultimately to deduce the sequence of amino acids in the protein. To establish the authenticity of the recombinant gene, this sequence can be compared to the amino acid sequence derived for the purified protein, isolated from human placenta (Schlaepfer, D.D., et al., Proc. Nat'l. Acad. Sci. USA, 84: 6078-6082 (1987)).

## EXAMPLE

### A. Construction of Human Placenta cDNA Library in Lambda gt11

Such a library is commercially available and was purchased from Clontech Laboratories, Inc., Palo Alto, California (catalog # HL1008) or can be constructed according to the procedures established in the art and readily available in this laboratory.

### B. Cloning of Endonexin II

To screen the human placenta library for endonexin II clones, the lambda gt11 Clontech library (approximately 1-2 x $10^5$ plaques) was plated on lawns of E. coli Y1090 and incubated at 42° for 4-5 hours. Plates were then overlaid with nitrocellulose filters which had previously been soaked in 10 mM isopropyl B-D-thiogalactopyranoside (IPTG) and the positions of the filters marked by a needle. The plates were then incubated overnight at 37° C. The next morning, filters were placed in blocking solution, 3% gelatin in TBS (20mM Tris, 500 mM NaCl, pH 7.5) for 45 minutes at room temperature, with gentle agitation. The filters were then placed in first antibody, a 1:50 dilution of rabbit anti-endonexin II in a solution containing 1% gelatin in TBS (antibody buffer). Filters incubated overnight at room temperature, with gentle agitation.

The next morning, filters were washed twice in TTBS (TBS containing 0.05% Tween 20) and once in TBS (10-15 minutes each) with gentle agitation. The filters were then placed in second antibody (a 1:1000 dilution of biotinylated goat anti-rabbit IgG in antibody buffer, Vector Laboratories, Burlingame, CA) for 1 1/2 hours at room temperature, with gentle agitation. The filters were then washed 3 times (20 minutes each) in TBS, with gentle agitation. This was followed by a 30 minute incubation in Vectastain ABC kit reagents (Vector Laboratories, Burlingame, CA). This was a premixed solution of 2 drops A (avidin DH) - 2 drops B (biotinylated horseradish peroxidase H) per 10 ml TBS. The filters were then washed 3 times in TBS (15 minutes each) and color development was carried out as follows:

1) 60 mg HRP color development reagent (Bio-Rad Laboratories, Richmond Ca) was added to 20 ml ice cold methanol.

2) Immediately prior to use, 60 ul of ice cold 30% $H_2O_2$ was added to 100 ml TBS.

The two solutions were added together and the filters were placed in the mixture. Color development took about 5 minutes, after which the filters were rinsed in distilled water and dried. (See Young, R.A. and Davis, R.W., Proc. Nat'l. Acad. Sci. USA, above, for further details).

The initial screen yielded 21 potentially positive clones for endonexin II. Each of the 21 putative clones, along with a negative control, was removed as an impure plug from its respective plate, replated at a density of about 5 X $10^3$ plaques per plate and rescreened with antibody, as described above. Upon rescreening, 8 of the original 21 clones remained positive. The 8 clones were designated as LC 3, 5, 6, 7, 8, 11, 12 and 16. Each of these (along with a negative control) was plated at high density ( ~ 2500 plaques/large plate) and low density ( ~ 250 plaques/large plate). All 8 positive clones were then plaque purified via another cycle of reaction with antibody; 5 of the 8 endonexin II clones (LC 3, 5, 8, 11, 16) gave strong signals with the antibody while 3 clones (LC 6, 7, 12) gave weaker signals suggesting that they might be negative.

In order to confirm the identity of the antibody positive clones as endonexin II, two oligonucleotides were designed based upon the known amino acid sequence of endonexin II (Schlaepfer, D.D., et al. above). The 30-mer

```
     F   N   I   R   K   E   F   R   K   N
5'  TTC AAC ATC AGA AAG GAG TTC AGA AAG AAC 3'
```

is found in a region of non-homology between lipocortins I, II, and endonexin II while the 41 mer

```
    L   K   G   A   G   T   N   E   K   V   L   T   E   I
5' CTG AAG GGG GCI GGA ACC AAC GAG AAI GTT CTI ACT GAG AT 3'
```

is located in a region of strong homology among the 3 proteins. Thus, hybridization with the 41-mer might identify all three proteins, while hybridization with the 30-mer (or with its inverse complement, as in this invention) would be specific for endonexin II. The eight antibody positive clones were hybridized to each oligonucleotide via Southern transfers as well as by probing DNA from plaques transferred onto nitrocellulose filters, as described below.

Size analyses of clones were made via restriction endonuclease digestions of purified DNA prepared from each antibody positive clone. This was done in duplicate. Following electrophoresis and ethidium bromide staining, the gels were placed in an alkaline solution and agitated in order to denature the DNA. Capillary transfer of DNA in the gel to Gene Screen Plus membranes was carried out overnight in the same solution. Following transfer, the membranes were neutralized and dried.

The 8 antibody positive clones were also plated at a density of approximately 250 plaques per plate on E. coli Y1090 and incubated for 4 hours at 42° C. The plates were refrigerated overnight and the next morning they were overlaid with nitrocellulose filters. The positions of the filters were marked with a needle. These filters were removed after 30 seconds and each was replaced by a second filter which was removed after several minutes. The duplicate filters were allowed to air dry and then prepared for hybridization as described in Maniatis, et al., supra. This involved DNA denaturation in 0.5 M NaOH, 1.5 M NaCl, neutralization in 1 M Tris-HCl, pH 7.5, 1.5 M NaCl, and 3 X SSC followed by heating of the filters for 2 hours at 80° C. in vacuo. Two sets of duplicate filters were prepared, one for each oligonucleotide.

Approximately 100 pmoles of each oligonucleotide were radioactively labelled by incubation with $^{32}$[P]-ATP and $T_4$ polynucleotide kinase, essentially as described by Maniatis, et al., supra. Southern blots and nitrocellulose filters, prepared as described above, were prehybridized at 45° C. overnight in a solution containing a 1:25 dilution of 5% nonfat dry milk (Blotto) in 5 X SSPE (1 X SSPE is 150 mM NaCl, 10 mM $NaHPO_4$, pH 7.2, 1 mM EDTA). The next morning, one Southern blot plus one set of filters were placed into each of two separate solutions containing either $^{32}$[P] 41-mer or $^{32}$[P] 30-mer (1.5-2.0 X $10^6$ cpm/ml of hybridization solution). Hybridization was carried out at 45° C. overnight. The next morning, unhybridized probe was removed by sequential washing at 42° C. in 2 X SSPE, 0.1% SDS (sodium dodecyl sulfate). After overnight exposure at -80° C., autoradiographs were developed. Results from the Southern blots indicated that clone 8 reacted positively with both the 30-mer and the 41-mer. Since the 30-mer oligonucleotide resides in a region of non-homology among the 3 proteins, this suggests that this clone ( λ LC8) contains all or part of the endonexin II coding region. The results from the nitrocellulose filters showed that plaques of clone 8 hybridized strongly to the 30-mer. The 41-mer hybridized weakly to λ LC8. This oligonucleotide also gave weak but positive results on the Southern blots.

The cDNA insert of λ LC8 was subcloned into the EcoRI site of M13mp18 and both strands were sequenced using the chain termination method. (Sanger, et al. Proc. Nat'l. Acad. Sci. USA, 74:5463-5467 (1977). Sequence analysis revealed that this clone encoded 317 amino acids of endonexin II, but lacked the N-terminal methionine as well as the poly(A) tail and polyadenylation signal. Using these sequence data, the following 38-mer, located near the 5′ end of the antibody positive clone, was synthesized:

5′ GAGCGGGCTGATGCAGAAACTCTTCGGAAGGCTATGAA 3′.

The oligonucleotide was kinased and the Clontech human placenta library was rescreened. Sixteen positive clones were identified and plaque purified. Since there is no internal EcoRI site in the original endonexin II cDNA clone ( λ LC8) and there exists a unique HincII site located approximately 40 bp downstream from the 3′ end of the 38-mer, DNA from each of the 16 new clones was restricted with EcoRI and HincII, run on a 1% agarose gel and transferred to nitrocellulose by Southern blotting. The blot was then probed using the $^{32}$P-labelled 38-mer prepared by kinasing. Those clones containing both the largest EcoRi-HincII fragments as well as the largest total sizes were chosen for screening. Four such clones were subsequently sequenced from both ends up to the 5′ and 3′ overlaps with the antibody-positive clone. Each of the four clones was found to extend into the 5′ untranslated region as well as to contain the poly(A) tail.

## DNA Sequence Determination

The complete sequence of human endonexin II DNA was obtained from the partial sequence of the antibody positive clone ( LC8) and the clones isolated from rescreening the Clontech human placenta library with the 38-mer. Purified DNA from each clone to be sequenced was restricted with EcoRI, and the cDNA insert isolated from a low melting point agarose gel, and subcloned into the EcoRI site of M13mp18. Transformations may be performed by using by using E. coli, e.g., E. coli of $pRK_6$ (E. coli designated as TG1),cells as hosts. In order to identify clones in both orientations for sequencing, the C-test procedure was employed as described by Hackett, et al., "An Introduction to Recombinant DNA Techniques", Benjamin Cummings Publishing Co., Menlo Park, CA (1984). Both strands were sequenced completely using the chain termination method. Protocols for preparing templates, as well as the sequencing reagents used throughout were those supplied with the Sequenase kit (United States Biochemical Corp.).

## Synthesis of Primers

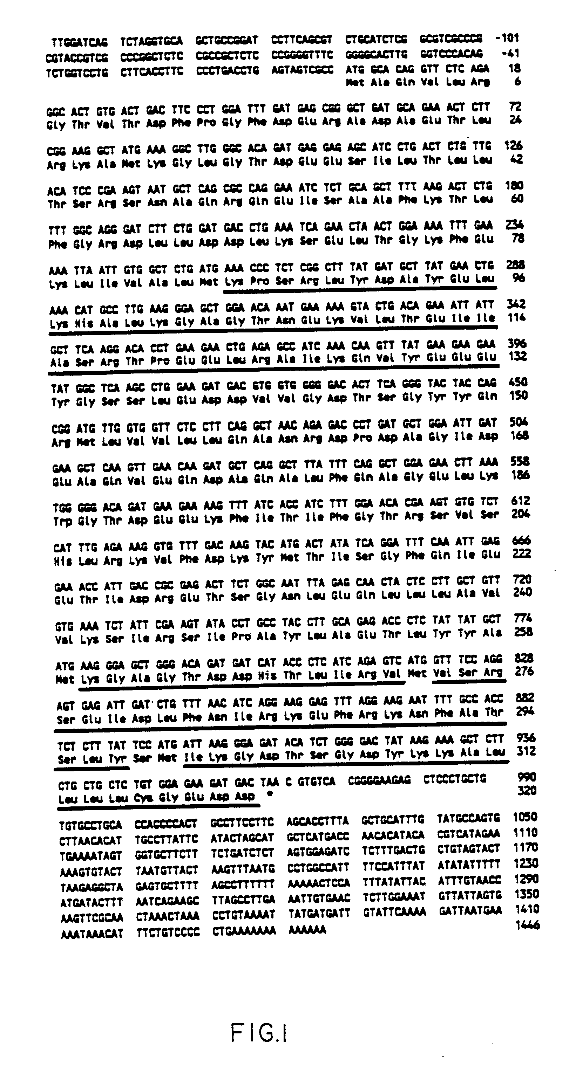

In addition to the universal primer supplied with the Sequenase kit, a series of oligonucleotides was synthesized as sequence data became available for both strands of the antibody positive clone. The oligomers were synthesized in an automatic DNA synthesizer (Model 380 A, applied Biosystems) using either methoxy or $\beta$ -cyanoethyl phosphoramidites. Below is a list of the oligomers synthesized. Oligomers 1-6 have the same sequence as the mRNA while oligomers 7-12 are complementary to the mRNA. The position of each oligomer in the cDNA sequence shown in Figure 1 is indicated.

| Synthetic Oligonucleotide Primers | | |
|---|---|---|
| Oligomer Sequence | Sequence (5′ to 3′) | Position in cDNA |
| 1 | GCTTATGAACTGAAACATGCC | 277-297 |
| 2 | GACCCTGATGCTGGAATTGATG | 484-505 |
| 3 | GAGACTTCTGGCAATTTAGAGC | 682-703 |
| 4 | CTGTTTAACATCAGGAAGGAG | 841-861 |
| 5 | CTGATCTCTAGTGGAGATCT | 1132-1151 |
| 6 | AGAGGCTAGAGTGCTTTTAGCC | 1233-1254 |
| 7 | AGATCTCCACTAGAGATCAG | 1131-1151 |
| 8 | CTCCTTCCTGATGTTAAACAG | 841-861 |
| 9 | CCTGATATAGTCATGTACTTGTC | 631-653 |
| 10 | GTCCCCCACCACGTCATCTTCC | 411-432 |
| 11 | GGCATGTTTCAGTTCATAAGC | 277-297 |
| 12 | TTCCTGGCGCTGAGCATTAC | 137-156 |

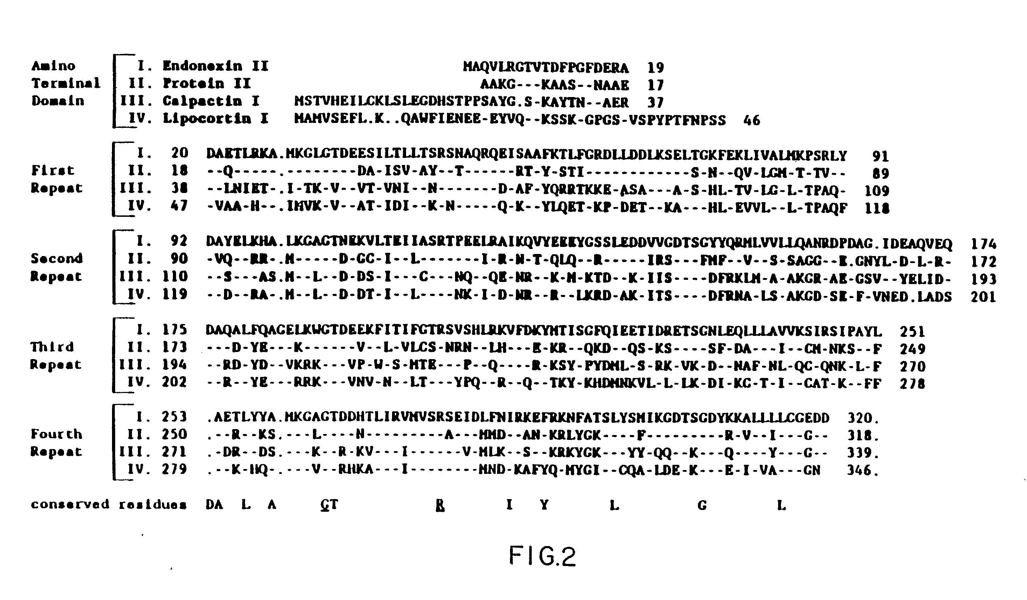

The complete nucleotide sequence of endonexin II cDNA is shown in Figure 1. The sequence identity between endonexin II and other members of the annexin family is shown in Figure 2.

## Northern Blot Analysis

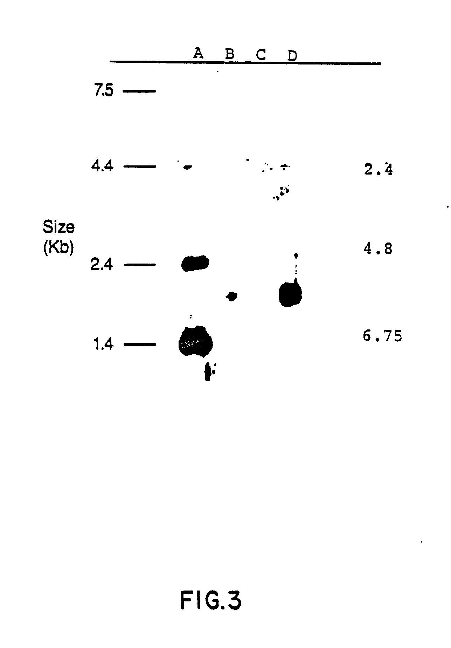

Formaldehyde gels were prepared as described by Davis, L.G., et al. in "Basic Methods in Molecular Biology", Elsevier Scientific Publishing Co., Inc., 52 Vanderbilt Avenue, New York, NY (1986). Samples containing 2µg or micrograms of poly(A)-containing RNA were heated at 95°C. for 2 min., quickly chilled on ice, and then heated for an additional 15 min. at 56°C. prior to loading. The gels were run overnight at 40 V. Transfers to Gene Screen Plus were carried out overnight in 10X SSC (1X SCC is 0.15 M NaCl, 0.015 M sodium citrate pH 7.0). Following transfer, blots were soaked in 2X SSC for 5 min., damp dried, and baked in a vacuum oven at 80°C. for 90 min. Prehybridization was done at 65°C. for 2-3 hr. in 5X SSPE, 1X Blotto (a 1:50 dilution of 5% nonfat dry milk), 0.25% SDS. Hybridization was carried out overnight at 65°C. in the same solution to which at least 1X $10^6$ cpm/ml of a $^{32}$P-labelled full length endonexin II clone was added. The probe was prepared either by nick translation or random priming. The blots were washed twice at room temperature, 15 min. each, in 2X SSPE, 0.25% SDS, twice more in the same solution, 15 min. each, at 65°C. and then twice in 1X SSPE, 0.25% SDS, 15 min. each, 65°C. After rinsing in 1X SSPE, room temperature, blots were air dried and autoradiographed. As shown in Figure 3, a single, approximately 1600 bp mRNA species was observed.

## Production of Recombinant Human Endonexin II

The cDNA insert of a full length endonexin II clone was removed from lambda gt11 by restriction with EcoRI and was purified from a low melting point agarose gel. Subsequent digestion with NcoI and HindIII resulted in a fragment which contained the entired coding region plus 323 bp of 3′ untranslated region. The ATG translation initiation codon is located within the NcoI site used. This fragment was force cloned between the NcOI and HindIII sites of the expression vector PKK233-2 (Pharmacia) which contains the hybrid tac promoter and carries the gene for ampicillin resistance. The lac $i^Q$ containing E. coli was then transformed with a recombinant plasmid.

Overnight cultures of the E. coli carrying either the recombinant plasmid or plasmid alone were grown at 37°C. in minimal medium plus 100 ug/ml ampicillin. These cultures were then used to innoculate LB containing 100 ug/ml ampicillin. The resultant cultures were grown at 37°C. until A550 = 0.3-0.5. IPTG was then added to a concentration of 3 mM. Duplicate cultures served as uninduced controls. The cells were grown for an additional 2 hr. at 37°C., harvested by centrifugation and frozen for subsequent assays. A Western blot employing rabbit anti-endonexin II serum is shown in Figure 4.

Other expression vectors which allow one to produce the protein encoded by the structural endonexin II gene include, but are not limited to, the following:
(1) Expression vectors for prokaryotic use include a number of commercially available systems using a variety of promoters (e.g, pL, pR, lac, and tac). (2) Eukaryotic systems are also either commercially available or proprietary systems developed in this laboratory may be used. These include bovine papilloma virus, Epstein-Barr virus, and a DHFR-selectable system in Chinese hamster ovary cells. Using the methodology developed to purify this protein from human placenta, recombinant derived protein can be purified from either prokaryotic or eukaryotic systems. Protein purification in this manner can be evaluated in vacuo and in vivo for its clinical efficacy in human diseases, including those human diseases characterized by inflammation which is mediated by the release of arachidonate. Arachidonate is the precursor for the biosynthesis of prostaglandins and leukotrienes both active in inducing inflammatory diseases. In addition, endonexin II may be useful in inhibiting other cellular events, such as coagulation, by binding to and interfering with normal phospholipid structure and/or function.

## Deposit of Strains Useful in Practicing the Invention

Deposits of biologically pure cultures of the strain for practicing this invention (designated pRK6) were made with the American Type Culture Collection, 12301 Parklawn Drive, Rockville, Maryland on March 21, 1989 under accession number ATCC 67916.

The deposit was made in accordance with the provisions of the Budapest Treaty. Said cultures will remain permanently available for a term of at least five years after the most recent request for furnishing of a sample, and in any case, 30 years after the date of the deposit. Should the culture become nonviable or be inadvertently destroyed, it will be replaced with a viable culture(s) of the same taxonomic description.

## Claims

1. Human endonexin II essentially free of other proteins of human origin.
2. Human endonexin II according to Claim 1 wherein the endonexin is in essentially pure form.
3. Human endonexin II according to Claims 1 or 2 wherein the endonexin is recombinant endonexin II.
4. Human endonexin II according to any of Claims 1 to 3 having the amino acid sequence described in Figure 1.
5. A process which comprises the expression of DNA encoding human endonexin II in a self-replicating, recombinant host system consisting of:

   A. preparing a replicable expression vector capable of expressing the DNA sequence encoding human endonexin II in a suitable self-replicating recombinant host system;

   B. transforming said host system to obtain a recombinant host system;

   C. maintaining said recombinant host system under conditions permitting expression of said endonexin II-encoding DNA sequence to produce human endonexin II; and

   D. recovering said human endonexin II.

6. The process of Claim 5 wherein the expression vector is a bacteriophage.
7. The process of Claim 12 wherein the bacteriophage is lambda gt10 or lambda gt11.
8. The process of Claim 7 wherein the expression vector is a plasmid.
9. The process of Claim 8 wherein the plasmid is derived from pBR322.
10. The process of Claim 7 wherein the plasmid is pRK6.
11. A composition comprising a therapeutically effective amount of human endonexin II according to any of Claims 1 to 4 in a mixture with a pharmaceutically acceptable carrier.
12. A cDNA formed by copying mRNA which mRNA is capable of directing the synthesis in a cell free protein synthesizing system which is immunologically reactive with antibodies directed against endonexin II proteins.
13. A vector comprising in recombinant form a cDNA according to Claim 12.
14. The vector according to Claim 13 wherein said vector is an expression vector.
15. The vector according to Claim 14 wherein the expression vector is capable of expressing the endonexin II of any of Claims 1 to 4.
16. A host cell transformed by the vector of any of Claims 13 to 15.

17. A transformed host cell according to Claim 16 which is obtained by transforming an E. coli, B. subtilis or eukaryotes.

TTGGATCAG TCTAGGTGCA GCTGCCGGAT CCTTCAGCGT CTGCATCTCG GCGTCGCCCG -101

CGTACCGTCG CCCGGCTCTC CGCCGCTCTC CCGGGGTTTC GGGGCACTTG GGTCCCACAG -41

TCTGGTCCTG CTTCACCTTC CCCTGACCTG AGTAGTCGCC ATG GCA CAG GTT CTC AGA 18

Met Ala Gln Val Leu Arg 6

GGC ACT GTG ACT GAC TTC CCT GGA TTT GAT GAG CGG GCT GAT GCA GAA ACT CTT 72

Gly Thr Val Thr Asp Phe Pro Gly Phe Asp Glu Arg Ala Asp Ala Glu Thr Leu 24

CGG AAG GCT ATG AAA GGC TTG GGC ACA GAT GAG GAG AGC ATC CTG ACT CTG TTG 126

Arg Lys Ala Met Lys Gly Leu Gly Thr Asp Glu Glu Ser Ile Leu Thr Leu Leu 42

ACA TCC CGA AGT AAT GCT CAG CGC CAG GAA ATC TCT GCA GCT TTT AAG ACT CTG 180

Thr Ser Arg Ser Asn Ala Gln Arg Gln Glu Ile Ser Ala Ala Phe Lys Thr Leu 60

TTT GGC AGG GAT CTT CTG GAT GAC CTG AAA TCA GAA CTA ACT GGA AAA TTT GAA 234

Phe Gly Arg Asp Leu Leu Asp Asp Leu Lys Ser Glu Leu Thr Gly Lys Phe Glu 78

AAA TTA ATT GTG GCT CTG ATG AAA CCC TCT CGG CTT TAT GAT GCT TAT GAA CTG 288

Lys Leu Ile Val Ala Leu Met Lys Pro Ser Arg Leu Tyr Asp Ala Tyr Glu Leu 96

AAA CAT GCC TTG AAG GGA GCT GGA ACA AAT GAA AAA GTA CTG ACA GAA ATT ATT 342

Lys His Ala Leu Lys Gly Ala Gly Thr Asn Glu Lys Val Leu Thr Glu Ile Ile 114

GCT TCA AGG ACA CCT GAA GAA CTG AGA GCC ATC AAA CAA GTT TAT GAA GAA GAA 396

Ala Ser Arg Thr Pro Glu Glu Leu Arg Ala Ile Lys Gln Val Tyr Glu Glu Glu 132

TAT GGC TCA AGC CTG GAA GAT GAC GTG GTG GGG GAC ACT TCA GGG TAC TAC CAG 450

Tyr Gly Ser Ser Leu Glu Asp Asp Val Val Gly Asp Thr Ser Gly Tyr Tyr Gln 150

CGG ATG TTG GTG GTT CTC CTT CAG GCT AAC AGA GAC CCT GAT GCT GGA ATT GAT 504

Arg Met Leu Val Val Leu Leu Gln Ala Asn Arg Asp Pro Asp Ala Gly Ile Asp 168

GAA GCT CAA GTT GAA CAA GAT GCT CAG GCT TTA TTT CAG GCT GGA GAA CTT AAA 558

Glu Ala Gln Val Glu Gln Asp Ala Gln Ala Leu Phe Gln Ala Gly Glu Leu Lys 186

TGG GGG ACA GAT GAA GAA AAG TTT ATC ACC ATC TTT GGA ACA CGA AGT GTG TCT 612

Trp Gly Thr Asp Glu Glu Lys Phe Ile Thr Ile Phe Gly Thr Arg Ser Val Ser 204

CAT TTG AGA AAG GTG TTT GAC AAG TAC ATG ACT ATA TCA GGA TTT CAA ATT GAG 666

His Leu Arg Lys Val Phe Asp Lys Tyr Met Thr Ile Ser Gly Phe Gln Ile Glu 222

GAA ACC ATT GAC CGC GAG ACT TCT GGC AAT TTA GAG CAA CTA CTC CTT GCT GTT 720

Glu Thr Ile Asp Arg Glu Thr Ser Gly Asn Leu Glu Gln Leu Leu Leu Ala Val 240

GTG AAA TCT ATT CGA AGT ATA CCT GCC TAC CTT GCA GAG ACC CTC TAT TAT GCT 774

Val Lys Ser Ile Arg Ser Ile Pro Ala Tyr Leu Ala Glu Thr Leu Tyr Tyr Ala 258

ATG AAG GGA GCT GGG ACA GAT GAT CAT ACC CTC ATC AGA GTC ATG GTT TCC AGG 828

Met Lys Gly Ala Gly Thr Asp Asp His Thr Leu Ile Arg Val Met Val Ser Arg 276

AGT GAG ATT GAT CTG TTT AAC ATC AGG AAG GAG TTT AGG AAG AAT TTT GCC ACC 882

Ser Glu Ile Asp Leu Phe Asn Ile Arg Lys Glu Phe Arg Lys Asn Phe Ala Thr 294

TCT CTT TAT TCC ATG ATT AAG GGA GAT ACA TCT GGG GAC TAT AAG AAA GCT CTT 936

Ser Leu Tyr Ser Met Ile Lys Gly Asp Thr Ser Gly Asp Tyr Lys Lys Ala Leu 312

CTG CTG CTC TGT GGA GAA GAT GAC TAA C GTGTCA CGGGGAAGAG CTCCCTGCTG 990

Leu Leu Leu Cys Gly Glu Asp Asp * 320

TGTGCCTGCA CCACCCCACT GCCTTCCTTC AGCACCTTTA GCTGCATTTG TATGCCAGTG 1050

CTTAACACAT TGCCTTATTC ATACTAGCAT GCTCATGACC AACACATACA CGTCATAGAA 1110

TGAAAATAGT GGTGCTTCTT TCTGATCTCT AGTGGAGATC TCTTTGACTG CTGTAGTACT 1170

AAAGTGTACT TAATGTTACT AAGTTTAATG CCTGGCCATT TTCCATTTAT ATATATTTTT 1230

TAAGAGGCTA GAGTGCTTTT AGCCTTTTTT AAAAACTCCA TTTATATTAC ATTTGTAACC 1290

ATGATACTTT AATCAGAAGC TTAGCCTTGA AATTGTGAAC TCTTGGAAAT GTTATTAGTG 1350

AAGTTCGCAA CTAAACTAAA CCTGTAAAAT TATGATGATT GTATTCAAAA GATTAATGAA 1410

AAATAAACAT TTCTGTCCCC CTGAAAAAAA AAAAAA 1446

FIG.1

```
Amino      I. Endonexin II                               MAQVLRGTVTDFPGFDERA  19
Terminal  II. Protein II                                     AAKG---KAAS--NAAE  17
Domain   III. Calpactin I     MSTVHEILCKLSLEGDHSTPPSAYG.S-KAYTN--AER  37
          IV. Lipocortin I    MAMVSEFL.K..QAWFIENEE-EYVQ--KSSK-GPGS-VSPYPTFNPSS  46

           I.  20  DAETLRKA.MKGLGTDEESILTLLTSRSNAQRQEISAAFKTLFGRDLLDDLKSELTGKFEKLIVALMKPSRLY  91
First     II.  18  --Q-----.---------DA-ISV-AY--T------RT-Y-STI-----------S-N--QV-LGM-T-TV--  89
Repeat   III.  38  --LNIET-.I-TK-V--VT-VNI--N-------D-AF-YQRRTKKE-ASA---A-S-HL-TV-LG-L-TPAQ-  109
          IV.  47  -VAA-H--.IMVK-V--AT-IDI--K-N-----Q-K--YLQET-KP-DET--KA---HL-EVVL--L-TPAQF  118

           I.  92  DAYELKHA.LKGAGTNEKVLTEIIASRTPEELRAIKQVYEEEYGSSLEDDVVGDTSGYYQRMLVVLLQANRDPDAG.IDEAQVEQ  174
Second    II.  90  -VQ--RR-.M-----D-GC-I--L-------I-R-N-T-QLQ--R-----IRS---FMF--V--S-SAGG--E.GNYL-D-L-R-  172
Repeat   III. 110  --S---AS.M--L--D-DS-I---C---NQ--QE-NR--K-M-KTD--K-IIS----DFRKLM-A-AKGR-AE-GSV--YELID-  193
          IV. 119  --D--RA-.M--L--D-DT-I--L----NK-I-D-NR--R--LKRD-AK-ITS----DFRNA-LS-AKGD-SE-F-VNED.LADS  201

           I. 175  DAQALFQAGELKWGTDEEKFITIFGTRSVSHLRKVFDKYMTISGFQIEETIDRETSGNLEQLLLAVVKSIRSIPAYL  251
Third     II. 173  ---D-YE---K------V--L-VLCS-NRN--LH---E-KR--QKD--QS-KS----SF-DA---I--CM-NKS--F  249
Repeat   III. 194  --RD-YD--VKRK---VP-W-S-MTE---P--Q----R-KSY-PYDML-S-RK-VK-D--NAF-NL-QC-QNK-L-F  270
          IV. 202  --R--YE---RRK---VNV-N--LT---YPQ--R--Q--TKY-KHDMNKVL-L-LK-DI-KC-T-I--CAT-K--FF  278

           I. 253  .AETLYYA.MKGAGTDDHTLIRVMVSRSEIDLFNIRKEFRKNFATSLYSMIKGDTSGDYKKALLLLCGEDD  320.
Fourth    II. 250  .--R--KS.---L----N---------A---MMD--AN-KRLYGK----F---------R-V--I---G--  318.
Repeat   III. 271  .-DR--DS.---K--R-KV---I------V-MLK--S--KRKYGK---YY-QQ--K---Q----Y---G--  339.
          IV. 279  .--K-HQ-.---V--RHKA---I--------MND-KAFYQ-MYGI--CQA-LDE-K---E-I-VA---GN  346.

conserved residues  DA  L  A     GT            R        I   Y         L           G        L
```

FIG.2

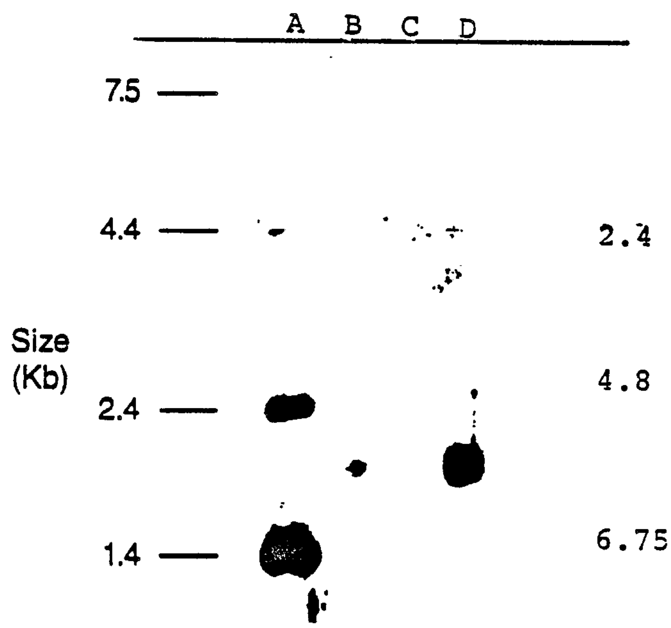


FIG.3